(19) Europäisches Patentamt
European Patent Office
Office européen des brevets

(11) Veröffentlichungsnummer: **0 263 528 B1**

(12) **EUROPÄISCHE PATENTSCHRIFT**

(45) Veröffentlichungstag der Patentschrift: **20.12.95**

(51) Int. Cl.[6]: **A61K 39/112**

(21) Anmeldenummer: **87114770.8**

(22) Anmeldetag: **09.10.87**

(54) **Salmonella-Lebendimpfstoffe mit Wirtsspezies-angepassten Attenuierungshöhen und antiepidemischer Potenz**

(30) Priorität: **10.10.86 DD 295161**
**10.10.86 DD 295162**
**10.10.86 DD 295160**

(43) Veröffentlichungstag der Anmeldung:
**13.04.88 Patentblatt 88/15**

(45) Bekanntmachung des Hinweises auf die
Patenterteilung:
**20.12.95 Patentblatt 95/51**

(84) Benannte Vertragsstaaten:
**CH DE FR GB IT LI NL**

(56) Entgegenhaltungen:
**DE-A- 2 843 295**

**JOURNAL INFECT DISEASES, Band 110, 1962, Seiten 192-203; M. HERZBERG: "Livingorganisms as immunizing agents against experimental salmonellosis in mice. I.Virulence of auxotrophic mutants"**

(73) Patentinhaber: **TAD Pharmazeutisches Werk GmbH**
**Heinz-Lohmann-Strasse 5**
**D-27472 Cuxhaven (DE)**

(72) Erfinder: **Linde, Klaus, Prof.Dr.**
**Fritz Austel-Str. 137**
**DDR-7030 Leipzig (DD)**
Erfinder: **Beer, Jörg, Dr.**
**Hänselweg 28**
**DDR-7030 Leipzig (DD)**
Erfinder: **Herrmann, Jürgen, Dr.**
**Str. d. Friedens 6**
**DDR-7201 Mölbis (DD)**
Erfinder: **Hottmann, Hans, Dr.**
**Geschwister Scholl-Str. 3**
**DDR-7200 Borna (DD)**
Erfinder: **Randhagen, Bärbel**
**Prinz Eugen-Str. 40**
**DDR-7030 Leipzig (DD)**

(74) Vertreter: **Siemons, Norbert, Dr.-Ing. et al**
**Postfach 30 24 30**
**D-20308 Hamburg (DE)**

EP 0 263 528 B1

WISS. Z. KARL-MARX-UNIV. LEIPZIG, Band 28, Nr. 1, 1979, Seiten 47-54; K. LINDEet al.: "Stabile hochimmunogene Salmonella-, Shigella- und Pasteurella-mutantenmit zwei unabhängig voneinander attenuierenden multationen"

siehe Beilage

**Beschreibung**

Die Erfindung betrifft einen Salmonella-Lebendimpfstoff mit einer der jeweiligen Wirtsspezies angepaßten Attenuierungshöhe und einer zusätzlichen antiepidemischen Potenz gegen Salmonella-Infektionen. Die zur aktiven Immunisierung des Menschen und von Nutztieren geeigneten Impfstoffe aus lebenden Erregern basieren auf Impfstämmen, deren

- Attenuierungshöhe einer Wirtsspezies-angepaßten Virulenzreduktion entspricht, welche einerseits die inapparente Infektion und andererseits die ausreichende (aber zeitlich begrenzte) Persistenz der Impfstämme im Wirtsgewebe als Voraussetzung für eine hohe Immunogenität garantiert,
- Stabilität (keine Rückmutation zum Wildstamm unter nicht-selektiven Feldbedingungen) durch Verwendung von in vitro nicht nachweisbar revertierenden Einmarkerstämmen bzw. bei Reversionshäufigkeiten $\leq 10^{-7}$ über den Einbau von weiteren unabhängig voneinander die Virulenz reduzierenden Mutationen garantiert wird,
- verminderte Ausscheidung und verkürzte Überlebenszeit in der Außenwelt die Wahrscheinlichkeit der Impfstamm-Infektkettenbildung stark reduziert. Diese wahlweise einzubauende zusätzliche antiepidemische Potenz (laut WHO-Definition - Wld.Hlth.Org.techn.Rep.Ser. (1972), No 500 - ein Attenuierungsmarker), die die Virulenz des Erregers bei parenteraler Applikation kaum oder nicht beeinflußt, resultiert aus einer funktionell veränderten (nicht auf R-Form-Mutation beruhenden) Permeabilitäts-Barriere der outer membrane, u.a. nachweisbar
  - an der hohen Sensibilität gegen Galle, Aniondetergenzien und Makrolid-Antibiotika nach selektiver Reversion zur Galle-, bzw. Galle- und Aniondetergens-Toleranz (um die orale Applikation nicht zu beeinträchtigen)
  - an der hohen Sensibilität gegen Aniondetergenzien und Makrolide oder
  - an der hohen Sensibilität nur gegen Makrolide.

In den Patentschriften DD 155 294; DD 218 836 A1; DD 235 828 A1 und DD 218 836 A1 wurde(n)

- einerseits der Stand der Technik dargelegt, welcher entsprechend der WHO-Information (ICP/BVM 008/S 6144B, Juli 1981) und den Empfehlungen des Symposiums "Enteric infections in man and animals: Standardization of immunological procedures", Dublin 1982 (in: Developments in Biological Standardization, Vol. 53, S.Karger, Basel 1963) z.Zt. noch gültig ist,
- andererseits Prinziplösungen zur Herstellung von Impfstamm-Mutanten vorgeschlagen, die durch Kopplung von zwei oder mehreren Markern sowie durch Verwendung eines antiepidemischen Markers die Sicherheitsforderungen der WHO erfüllen.

Die hohe Immunogenität solcher potentieller Impfstämme beruht auf ihrer restlichen, unterhalb des klinischen Schwellenwertes liegenden Vermehrung im Wirt und der damit verbundenen ausreichenden, aber zeitlich begrenzten Persistenz im Gewebe.

Ihre epidemiologisch-epizootologische Unbedenklichkeit basiert auf einer durch Mutation veränderten Permeabilität der outer membrane, erkennbar u.a. an einer Makrolidantibiotika- und Tensidhypersensibilität, wodurch die Keime vermindert ausgeschieden werden und in der Außenwelt progressiv absterben, so daß Infektketten in der Regel nicht zustande kommen können. Die Mutation zur high sensitivity to tensides (Hst) - welche das parenterale Virulenzverhalten nicht bzw. nur gering beeinflußt - verhindert als antiepidemischer Marker die Ausbildung von Impfstamm-Infektketten durch eine

- verminderte Ausscheidung mit Faeces infolge der hohen Sensibilität u.a. gegen Galle und der hieraus bereits im Darmlumen eintretenden Inaktivierung und
- verkürzte Überlebenszeit der ausgeschiedenen Impfstammbakterien in der Außenwelt als Folge einer fehlenden Permeabilitäts-Barriere in der outer membrane gegen Tenside und andere Noxen.

Einzelheiten zum Stand der Technik, einschließlich der galE-Mutation, den Sm-id-Mutanten sowie den Aro⁻-Mutanten finden sich in den eingangs zitierten Patentschriften, den Materialien des Symposium "Enteric infections in man and animals: Standardization of immunological procedures", Dublin, 1982 (in: Developments in Biological Standardization, Vol. 53, S.Karger, Basel 1983) und folgenden Publikationen:

- Linde, K., Zbl. Bakt. Hyg., I.Abt.Orig.A 249, 203-214 (1981),
- Linde, K., Zbl. Bakt. Hyg., I.Abt.Orig.A 249, 350-361 (1981),
- Linde, K., Wonitzki, Ch., Randhagen, B., Zbl. Bakt. Hyg., I.Abt.Orig.A 250, 478-489 (1981),
- Linde, K., Arch.exper.Vet.med. 36, 657-772 (1982),
- Linde, K., Wonitzki, Ch., Beer, J., Randhagen, B., Keller, H.-P., Z. ges. Hyg.30, 141-147 (1984),
- Linde, K., WHO working paper VPH/SPSC/WP/86,7
- Levine, M.M., WORLD HEALTH, April 1986, IX ISSN 0043-8502, p. 24-26
- Brown, M.R., Williams, P., Ann. Rev. Microbiol. 39, 527-567 (1985),
- Hancock, R.E.W., Ann. Rev. Microbiol. 38, 237-264 (1984),

- WHO/CDD/86,15 Diarrhoeal Disease Control Programme p. 12-13
- WHO/CDD/86,16 Control of Diarrhoeal Diseases p. 71-73.

Die als Stand der Technik oben zitierten Prinziplösungen zur Herstellung von Lebendimpfstoffmutanten weisen folgende Nachteile und deren Ursachen auf:

1.) Mutationen zur Pur⁻-(gilt nicht für alle Wirtsspezies) bzw. Aro⁻-Auxotrophie, deren Attenuierungsgrad vom Metabolitenmangel in vivo abhängt, sowie galE-Mutanten, bei denen es in vivo infolge einer Anreicherung der UDP-Galaktose zu einer Galaktolyse kommt, gestatten lediglich die Herstellung von hochattenuierten, im Wirt nur gering sich vermehrenden bzw. unzureichend persistierenden und daher die zelluläre Immunität zeitlich kürzer als möglich stimulierenden Einmarker-Stämmen. Dies gilt besonders für S.typhimurium-Pur⁻-Lebendimpfstoffmutanten zur Anwendung für Kälber, bei denen - infolge vollständigen Fehlens von nicht-phosphorylierten Purinen - solche Mutanten sich an der Grenze zur Überattenuierung bewegen. Die Einführung eines zweiten virulenz-reduzierenden Markers zwecks Garantierung der WHO-Sicherheits- bzw. Stabilitätsordnungen, führt in der Regel zur Überattenuierung.

2.) Die Verwendung des antiepidemischen Hst-Markers vermittelt zwar einerseits eine Unterbrechung von Infektketten (laut WHO-Definition zugleich Attenuierungsmarker), die gleichzeitige Galle-Sensibilität verlangt jedoch andererseits bei oraler Immunisierung eine ~10-fach höhere Dosierung, welche bei Dosen ≥10¹⁰ Keimen/Impfung mit der Gefahr von endotoxischen Nebenwirkungen und höheren Kosten verbunden sein kann.

3.) Bei Verwendung des Prinzips: Virulenzreduzierung über Stoffwechseldrift-Mutationen (Mutationen in essentiellen Enzymen und Stoffwechselkompartimenten: Selektion über Resistenz gegen Antibiotika und andere Noxen) ergeben sich de facto unbegrenzte Möglichkeiten zur Herstellung von Zwei- oder Dreimarker-Impfstämmen in jeder gewünschten - d.h. der jeweiligen Wirtsspezies angepaßten optimalen - Gesamt-Attenuierungshöhe und damit hoher Immunogenität.

Ein bedingter Nachteil dieser Lösung besteht jedoch darin, daß die Stabilitätsprüfung solcher Marker lediglich auf indirektem Wege durch den Nachweis der unveränderten Resistenz- und Attenuierungsparameter (nach ≥10 Nährböden- und/oder Tierpassagen) möglich ist.

Die Aufgabe der Erfindung besteht darin, einen Salmonella-Lebendimpfstoff mit antiepidemischen Eigenschaften zu schaffen, dessen Attenuierungshöhe durch die Art der Applikation nicht beeinträchtigt wird, so daß eine ausreichende Immunisierung durch übliche Dosierungen erreicht wird. Weiterhin ist es die Aufgabe der Erfindung, ein Verfahren zur Herstellung derartiger Lebendimpfstoffe anzugeben.

Zur Lösung dieser Aufgabe werden in dem unabhängigen Anspruch 1 ein Salmonella-Lebendimpfstoff, in dem unabhängigen Anspruch 3 ein Verfahren zur Herstellung von Salmonella-Lebendimpfstoffen und in dem unabhängigen Anspruch 6 die Verwendung derartiger Salmonella-Lebendimpfstoffe vorgeschlagen.

Die Aufgabe wird durch einen Salmonella-Lebendimpfstoff mit einer Wirtsspezies-angepaßten Attenuierungshöhe und mit einer antiepidemischen Potenz gemäß Anspruch 1 gelöst, der erfindungsgemäß einen aus Hst-Klonen gewonnenen Galle-toleranten revertanten (Rbt-Stamm) oder einen aus Rbt-Klonen gewonnenen Tensid-toleranten revertanten (Rtt-Stamm) Salmonella-Lebendimpfstamm enthält.

Mit einem Rbt- (reversion to bile tolerance) und einem Rtt-(reversion to tenside tolerance) Marker werden zwei weitere antiepidemische Marker zur Verfügung gestellt, mit denen die Impfstämme zur Herstellung von Lebendimpfstoffen versehen werden können. Sie besitzen gegenüber dem bekannten antiepidemischen Hst-Marker aufgrund von Veränderungen in der Outer-Membrane des Impfstammes andere vorteilhafte Eigenschaften. Dadurch sind beide Marker tolerant gegenüber Galle und können ohne Reduktion des Impfeffektes auch oral (mit etwa der gleichen Dosis wie der selbe Impfstamm ohne antiepidemischen Marker) appliziert werden. Der Rtt-Marker ist zusätzlich tolerant gegenüber Tenside. Beide Marker besitzen weiterhin ausreichende antiepidemische Potenz durch eine hohe Sensibilität gegenüber Makroliden und anderen Noxen.

In weiterer vorteilhafter Ausgestaltung des erfindungsgemäßen Lebendimpfstoffes enthält der Lebendimpfstoff als weitere Marker mindestens einen virulenzreduzierenden Stoffwechseldrift-Marker und/oder einen virulenzreduzierenden Marker Asparaginsäure-Auxotrophie.

Unter Verwendung dieser weiteren Marker kann durch deren gezielte Kombination die Attenuierungshöhe des Impfstammes optimal der jeweiligen Wirtsspezies angepaßt werden.

Als Impfstämme finden beispielsweise Verwendung:

- Salmonella typhimurium als Ein-, Zwei- oder Dreimarker-Impfstamm, welche am i.p.-Mäusemodell de facto alle Attenuierungshöhen umfassen, und zwar $LD_{50}$-Werte von ≤10⁴ bis ≥10⁸ (Wildstamm ≤10¹) Keimen,
- Salmonella typhi als vorzugsweise Drei- oder Mehrmarker-Impfstamm, welche am i.p.-Mäusemodell de facto alle Attenuierungshöhen umfassen, und zwar nach Injektion von 10⁶ Keimen eine Persistenz (Nachweis)-Dauer in Leber und Milz von ≥9 bis 2 (Wildstamm ˜19) Tagen aufweisen.

4

Davon ausgehend sind insbesondere folgende Varianten bzw. Kombinationen praxisrelevant, wobei das Prinzip auf alle Bakterien anwendbar ist (Asp⁻-Asparaginsäure-Auxotropie-Marker; Stwd - Stoffwechseldrift-Marker; Aep - antiepidemischer Marker):

| evt. Einmarker | Zweimarker | Dreimarker | Viermarker[**] |
|---|---|---|---|
| . Asp⁻ | . Asp⁻/Stwd | . Asp⁻/Stwd/Aep | |
| . Stwd | . Stwd/Stwd | . Asp⁻/Stwd/Stwd | |
| . Aep[*] | . Asp⁻/Aep | . Stwd/Stwd/Aep | |
| | . Stwd/Aep | . Stwd/Stwd/Stwd | |

[*]    in heterologem weniger virulentem Serotyp oder spontan vermindert virulentem Stamm

[**]    Viermarker etc. –    nach gleichem Konzept, im Prinzip aber nur von theoretischem Wert

In Realisierung dessen stehen für das Lösungsprinzip die folgenden Impfstämme beispielhaft.

| Salmonella typhi Ty 2 (S.Tg 2) und Salmonella typhimurium (S. tm.) | | | Labor-Nummer | Hinterlegungs-Nummer |
|---|---|---|---|---|
| | Ein-Marker | (Zwei- Drei- (Impf)-Stamm | (Vier- | |
| S.Ty 2 | Hat 6 | | | 3605 | ZIMET 11211 |
| S.Ty 2 | Rbt 31 | | | 3874 | ZIMET 11209 |
| S.Ty 2 | Rbt 32 | | | 3875 | ZIMET 11212 |
| S.Ty 2 | Hst 6 | Asp⁻ 6 | | 3991 | ZIMET 11213 |
| S.Ty 2 | Hst 6 | Asp⁻11 | | 3993 | ZIMET 11210 |
| S.Ty 2 | Hst 6 | Asp⁻15 | | 3995 | ZIMET 11214 |
| S.Ty 2 | Hst 6 | Asp⁻ 6 Rif 9 | | 4019 | ZIMET 11215 |
| S.Ty 2 | Rbt 1 | Asp⁻ 6 Rif 9 | Nal 18 | 4139 | ZIMET 11217 |
| S.Ty 2 | Rbt 2 | Asp⁻ 6 Rif 9 | Nal 1 | 4062/I | ZIMET 11216 |
| S.tm. | Asp⁻10 | | | 3796 | ZIMET 11218 |
| S.tm. | Asp⁻15 | | | 3801 | ZIMET 11219 |
| S.tm. | Asp⁻23 | | | 3805 | ZIMET 11208 |
| S.tm. | Asp⁻10 | Hst 29 | | 4011 | ZIMET 11220 |
| S.tm. | Asp⁻10 | Rbt 28 Nal 19 | | 4016 | ZIMET 11222 |
| S.tm. | Asp⁻10 | Hst 18 Rif 24 | | 4053 | ZIMET 11224 |
| S.tm. | Asp⁻10 | Hst 29 Rif 10 | | 4054 | ZIMET 11223 |
| S.tm. | Asp⁻10 | Rbt 28 | | 4014 | ZIMET 11221 |

Die Erfindung beschränkt sich nicht nur auf die Schaffung von Salmonella-Lebendimpfstoffen. Ein weiteres Ziel besteht darin, ein Verfahren zur Herstellung derartiger Impfstoffe anzugeben.

Dieses Ziel wird mit einem Verfahren mit den im Anspruch 3 angegebenen Merkmalen erreicht. Unter Verwendung von Gallehaltigem Nähragar werden mittels spontaner Mutation aus Makrolid-, Galle- und Anion-Detergent-sensitiven Hst-Klonen Galle-tolerante Rbt-Klone isoliert. Sollen gegebenenfalls Rtt-Marker eingesetzt werden, so wird das Verfahren unter Verwendung der erhaltenen Rbt-Klone fortgeführt, indem unter Verwendung von Anion-Detergent-haltigem Nähragar Tensidtolerante Rtt-Klone isoliert werden. Anschließend werden die erhaltenen Rbt- bzw. Rtt-Klone in immunogene Impfstämme oder in einen für die Impfstamm-Entwicklung vorgesehenen Wildstamm oder in als Zwischenprodukt vorliegende Ein-, Zwei- oder Mehrmarker-Impfstämme mit graduell abgestufter Attenuierung eingebaut. Aus den erhaltenen Stämmen werden mittels an sich bekannter Verfahren Lebendimpfstoffe hergestellt.

Das angegebene Verfahren läßt sich weiter vorteilhaft ausgestalten, wozu auf die Ansprüche 4 und 5 verwiesen wird. Für die Isolierung von Rbt-Mutanten ist es günstig, wenn Hst-Klone von Wild- und Impfstämmen 20 Stunden auf Nähragar kultiviert werden. Anschließend wird in physiologischer Kochsalzlösung suspendiert und diese Suspension auf eine Keimzahl von $10^{10}$ und $10^9$ Keime/ml eingestellt. Von den eingestellten Suspensionen werden 0,1 ml auf Nähragar mit 5000 $\mu$l Natrium-Desoxycholat/ml ausgespatelt. Der beimpfte Nähragar wird 48 Stunden bei 37°C bebrütet. Die gewachsenen Kolonien werden nunmehr auf einem Nähragar-Plattensatz mit Konzentrationen von

- 6 und 12 $\mu$g Oleandomycin/ml
- 600, 1200 und 2500 $\mu$g Natrium-Dodecylsulfat/ml
- 5000 und 10000 $\mu$g Natrium-Desoxycholat/ml

geimpft, wobei die Rbt-Stämme ein Toleranz- bzw. Sensibilitätsverhalten aufweisen, das auf 5000 und 10000 $\mu$g Natrium-Desoxycholat/ml Nähragar zu einem Wachstum führt und auf 12 $\mu$g Oleandomycin/ml Nähragar sowie 1200 $\mu$g Natrium-Dodecylsulfat/ml Nähragar durch fehlendes Wachstum gekennzeichnet ist. Für die Isolierung von Rtt-Mutanten erweist sich ein Verfahren als vorteilhaft, bei dem Rbt-Klone mit $10^9$ und $10^8$ Keime dieses Stammes auf Nähragar mit 10000 $\mu$g Natrium-Dodecylsulfat/ml gespatelt werden. Die gewachsenen Kolonien werden anschließend auf Nähragar mit 10000 und 20000 $\mu$g Natrium-Dodecylsulfat/ml und Nähragar mit 12 $\mu$g Oleandomycin/ml geimpft, wobei die Rtt-Stämme auf dem Detergent (und Galle)-Medium, jedoch nicht auf dem Makrolid-Antibiotika-Medium wachsen.

Anspruch 6 schließlich betrifft die Verwendung derartiger Salmonella-Lebendimpfstoffe zur Immunisierung von Menschen und Nutztieren gegen Salmonella-Infektionen.

Der erfindungsgemäße Impfstoff hat gegenüber den bekannten Impfstoffen den Vorteil, daß die neuen antiepidemischen Marker

- wie der Hst-Marker keinen nennenswerten Einfluß auf das parenterale Virulenzverhalten haben,
- im Gegensatz zum Hst-Marker nicht mit der Immunität bei oraler Impfstoffapplikation in Folge der restaurierten Galletoleranz interferieren,
- dem Impfstamm eine nennenswerte antiepidemische Potenz infolge einer reduzierten Ausscheidung und der verkürzten Überlebensfähigkeit in der Außenwelt belassen, wodurch die Gefahr der Impfstamm-Infektkettenbildung beseitigt oder vermindert wird, und
- als zusätzlicher Attenuierungsmarker die Stabilität -bezogen auf ausgewählte Klone- der Impfstämme um ≦$10^{-7}$ erhöhen.

Neben den bereits ausführlich beschriebenen Impfstämmen, die einen oder mehrere Stoffwechseldrift-Marker enthalten, hat sich gezeigt, daß sich Asp⁻-Marker (differente Genotypen mit unterschiedlicher Attenuierungshöhe) als solche oder in Kombination mit den vorgeschlagenen antiepidemischen und Stoffwechseldrift-Mutationen als vorteilhaft erweisen.

- Asparaginsäure-Auxotrophie mit (Klon-bzw. Genotyp-abhängiger) schwacher, mäßiger oder hoher Attenuierung In der Literatur finden sich lediglich drei Hinweise pauschalen Charakters zum Einfluß der Asparaginsäure-Auxotrophie auf die Virulenz:
  - Bacon, G.A., Burrows, T.W., Yates, M., Brit. J. exp. Path. 31, 714-724 (1950) Drei Asp⁻-Mutanten von Salmonella typhi sind für Mäuse mäßig attenuiert.
  - Bacon, G.A., Burrows, T.W., Yates, M., Brit. J. exp. Path. 32, 85-96 (1951) Injektion von Asparaginsäure senkt bei Mäusen die Letaldosis von S.typhi Asp⁻.
  - Herzberg, M., J. Inf. Dis. 110, 192-203 (1962) Von 7 Salmonella typhimurium-Asp⁻-Mutanten sind für Mäuse 1 Klon virulent, 3 (offensichtlich) gering und 3 mäßig attenuiert.

Eine Verwendung dieser durch die Asparaginsäure-Auxotrophie vermittelten Attenuierung wurde bislang nicht beschrieben, da offenbar das Vorurteil geprägt wurde, daß die Nutzung dieses Attenuierungsprinzipe für die Herstellung von Lebendimpfstoffen ungeeignet ist, weil die

  - mitgeteilten Attenuierungshöhen vermutlich die (irrtümliche) Vorstellungen vermittelten, daß die Asp⁻-Einmarker-Impfstämme zu virulent sind,
  - Markerkopplungen zwecks Erhöhung der Stabilität und Anhebung der Attenuierung steht erst seit wenigen Jahren und praxisrelevant erstmals durch uns zur Diskussion
  - meisten Mutanten hohe Reversionsraten zeigen (trotz - wie eigene Ergebnisse zeigen - Beibehaltung der Attenuierung [offensichtlich Suppressormutanten, vergleichbar der Reversion von Streptomycin-Dependenz zur Independenz]) und über das Vorkommen virulenter Asp⁻-Klone berichtet wurde.

Wir konnten feststellen, daß Asp⁻-Mutanten von Salmonella typhimurium - geprüft wurden neun Klone mit einer Reversionshäufigkeit von ≦$10^{-8}$ - in ein Spektrum von Stammen mit unterschiedlich hoher Virulenzreduktion zerfallen (siehe Tabelle 1, Seite 25)

Diese neun getesteten Asp⁻-Klone lassen sich unter Vorbehalt in wenigstens drei Attenuierungsgruppen unterteilen:

| Gruppe I : | $LD_{50}$ (Maus, i.p.) | $10^{2,3}$ Keime = 1 Stamm |
|---|---|---|
| Gruppe II : | $LD_{50}$ (Maus, i.p.) | $10^5$ Keime = 4 Stämme |
| Gruppe III: | $LD_{50}$ (Maus, i.p.) | $10^7$ Keime = 4 Stämme |
| Wildstamm: | $LD_{50}$ (Maus, i.p.) | $\leq 10^1$ Keime |

Diese drei Gruppen sind untereinander mittels Transduktion - die Versuche wurden mit den Klonen Asp⁻23, Asp⁻12 und Asp⁻15 (siehe Tabelle 1,Seite 25) durchgeführt - unter Verwendung des Phagen P22 zur Prototrophie rekombinierbar. Mit bedingter Kongruenz hierzu verhalten sich sieben von uns getestete Salmonella typhi-Hst/Asp⁻-Klone - mit einer in vitro nicht nachweisbaren bzw. $\leq 10^{-8}$ betragenden Reversion zur Prototrophie - hinsichtlich ihres Persistenzverhaltens in Mäusen sowie der mittleren Keimzahl im Leber- und Milz-Homogenat am 5. Tag nach Infektion mit i.p. $10^6$ Keimen (s. Tabelle 2, Seite 26)- :Attenuierungsgruppe I - 1 Stamm, II - 5 Stämme, III - 1 Stamm.

Zur Herstellung von Einmarker-Impfstämmen (stabile Asp⁻-Deletionsmutanten) oder Mehrmarker-Impfstämmen (zwecks Modifikation der Attenuierungshöhe und Erhöhung der Stabilität über Stoffwechseldrift-Mutationen und/oder Einführung einer antiepidemischen Potenz) mit einer der jeweiligen Wirtsspezies angepaßten Attenuierungshöhe (wobei auch zu berücksichtigen ist, daß die differente Empfänglichkeit von Kalb, Ferkel und Kücken gegenüber z.B. S.typhimurium für den Impfstamm Wirtsspezies-bezogen eine unterschiedlich hohe Virulenzreduktion erfordert) kann daher auf entsprechende Asp⁻-Klene mit unterschiedlich hoher Virulensreduktion - wie z.B. S.typhimurium Asp⁻10, Asp⁻23 oder Asp⁻15 (siehe Tabelle 1, Seite 25) oder S.typhi Hst/Asp⁻6, Hst/Asp⁻15 oder Hst/Asp⁻5 (siehe Tabelle 2, Seite 26) - zurückgegriffen werden. Der unter Beispielen - neben der Isolierung von stabilen Asp⁻-Einmarker-Impfstämmen differenter Attenuierungshöhen - beschriebene Aufbau von zwei- oder Drei- bzw. evtl. Viermarker-Impfstämmen unter Verwendung des Klen S.typhimurium Asp⁻10 und Klen S.typhi Hst/Asp⁻6 gilt sinngemäß für alle Asp⁻-Klone (Genetypen) mit geringerem, gleichen oder höherem Attenuierungsgrad.

Die Stabilität der Asp⁻-Marker ist mit direkten Methoden bestimmbar.

Es wurde bereits auf die Verwendung von Asp⁻-Mutanten in Verbindung mit Stoffwechseldrift-Mutanten verwiesen, wie z.B. der RNA-Polymerase- oder oder Gyrase [+])(DD 155 294 A1), welche Klen (Genetyp)-spesifisch den Attenuierungsgrad der Asp⁻-Einmarker-Impfstämme bzw. der Asp⁻/Hst bzw. Rbt-Zweimarker-Impfstämme (siehe oben) auf die gewünschte, d.h. Wirtsspezies angepaßte, Gesamtattenuierungshöhe anheben (siehe unter Ausführungsbeispiele).

Bei Einsatz von Asp⁻-Deletionsmutanten (repair des Defektes in vivo theoretisch möglich, aber unter nichtselektiven Feldbedingungen auszuschließen) als Einmarker-Impfstämme; sowie bei Kopplung von mit einer Häufigkeit $\leq 10^{-7}$ revertierenden Asp⁻-Markern mit antiepdiemischen und/oder Stoffwechseldrift-Markern(siehe oben) erhält man Impfstämme für den Einsatz als Lebendimpfstoffe, die

- unter nicht-selektiven Feldbedingungen als Asp⁻-Deletionsmutanten stabil sind sowie bei Zwei- oder Drei- und evtl. Viermarkerstämmen mit revertierenden Mutationen über eine Gesamtstabilität von $\leq 10^{-14}$ oder $\leq 10^{-21}$ bzw. evtl. $\leq 10^{-28}$ verfügen,
- infolge einer Wirtsspezies-angepaßten ausgewogenen Attenuierung immunogen sind und
- durch Verwendung eines antiepidemischen Markers nicht oder vermindert zur Impfstamm-Infektkettenbildung befähigt sind,

so daß die nach diesem Prinzip hergestellten Impfstämme die WHO-Forderung noch Stabilität, Immunogenität und verminderter Übertragungsfähigkeit vollauf erfüllen.

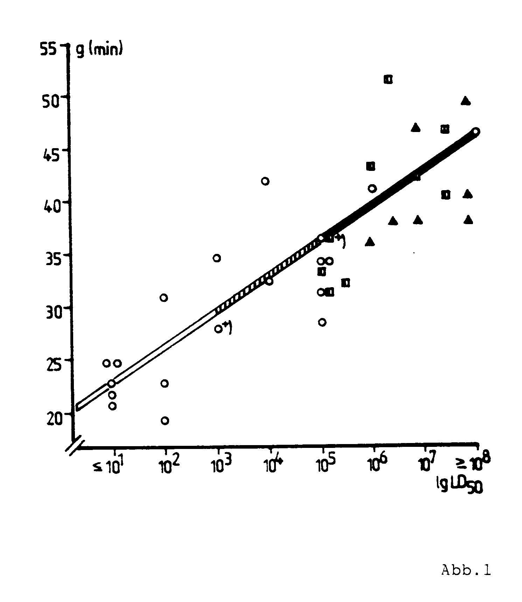

Bei dem Prinzip Stoffwechseldrift besteht eine Korrelation zwischen der Generationszeit (g) und dem Logarithmus der $LD_{50}$ (Maus i.p.) von S.typhimurium-Stoffwechseldrift-Mutanten, gezeigt in Abbildung 1 am Beispiel von attenuierten Nalidixinsäure-Resistenz-(Gyrase)-Klonen als ersten Marker (0), Rifampicin-Resi-

[+])Ebenso vorteilhaft ist die Verwendung entsprechender Klone (Genetypen) mit mutierten z.B. Ribosomenproteinen (u.a. Streptomycin-"Resistenz") oder anderer chromosomaler "Noxen-Resistenzen". Solche Marker-Kombinationen, einsetzbar z.B. anstelle des hinterlegten Stammes 4062/I - zwecks einfacher Unterscheidung der verschiedenen Impfstämme - wurden unter den Salmonella typhi Hinterlegungsstämmen nicht aufgeführt, um zweifelsfrei zu zeigen, daß mit Hilfe differenter Klone (Genetypen) degsleichen (z.B. Gyrase) "Resistenz"-Phänotyps unterschiedliche Attenuierungshöhen eingestellt werden können. Diese Prinzipien der Stoffwechseldrift sind offensichtlich auf alle Bakterien (z.B. Shigellen, Listerien) anwendbar.

stenz-(RNA-Polymerase)-Klonen als zweiten Marker (□) und Streptomycin-Resistenz-(Ribosomenprotein)-Klonen als dritten Marker (▲). Die Zweimarker-Mutanten leiten sich ab von einem Einmarkerstamm geringer Virulenzreduktion ($0^+$), die Dreimarkerstämme von einem Zweimarkerstamm mittlerer Virulenzreduktion (□$^+$).

Die nachfolgend angeführten Asp$^-$-Einmarker-Impfstämme (Reversion in vitro $\leq 10^{-8}$ bzw. nicht nachweisbar) und Zwei- bzw. Dreimarker-Impfstämme unter Verwendung von antiepidemischen (und Stoffwechseldrift)-Markern mit einer Einzel-Reversionshäufigkeit von $\leq 10^{-7}$, wie z.B. Salmonella typhimurium (S.tm.)

| - S.tm. Asp$^-$23 | LD$_{50}$ (Maus, i.p.)~$10^{2,3}$ | Keime |
|---|---|---|
| - S.tm. Asp$^-$10 | LD$_{50}$ (Maus, i.p.)~$10^{4,3}$ | Keime |
| - S.tm. Asp$^-$15 | LD$_{50}$ (Maus, i.p.)~$10^{6,7}$ | Keime |

sowie von Asp$^-$-Klonen dieser Attenuierungshöhe aufgebaute Mehrmarker-Mutanten, wie z.B.

| - S.tm. | Asp$^-$10/Hst oder Rbt | LD$_{50}$ (Maus, i.p.)~$10^5$ | Keime |
|---|---|---|---|
| - S.tm. | Asp$^-$10/Rbt/Nal 19 | LD$_{50}$ (Maus, i.p.)~$10^6$ | Keime |
| - S.tm. | Asp$^-$10/Hst/Rif 10 | LD$_{50}$ (Maus, i.p.)~$10^{6,5}$ | Keime |
| -S.tm. | Asp$^-$10/Hst/Rif 24 | LD$_{50}$ (Maus, i.p.)~$10^7$ | Keime |

bzw. die aus dem Zweimarker-Stamm S.typhi Ty 2 Hst/Asp$^-$6 entwickelten Impfstämme(Drei- und Vermarkerstämme)mit abgestufter Attenuierungshöhe, wie z.B. S.typhi Ty 2 (S.Ty 2)
- S.Ty 2 Hst 6 Asp$^-$6 Rif 9 Persistenzdauer ($10^6$ Keime/Maus i.p.): 9 Tage
  Mittlere Keimzahl (KZ) am 5. Tag in Leber-Milz-Homogenat: $2 \times 10^2$
- S.Ty 2 Asp$^-$6 Rif 9 Nal 1 Persistenzdauer: 9 Tage KZ : $10^1$
- S.Ty 2 Asp$^-$6 Rif 9 Nal 18 Persistenzdauer: 3 Tage KZ : $10^1$

welche als Beispiel stehen für alle nach gleichem Prinzip hergestellten Zwei-, Drei- und Viermarker-Impfstämmen jeder gewünschten Attenuierungshöhe sind zur Herstellung von hochimmunogenen Lebendimpfstoffen nach an sich bekannten Verfahren geeignet.

Die Erfindung soll nachstehend anhand folgender Ausführungsbeispiele näher erläutert werden.

Verwendete Stämme

- Salmonella typhimurium Wildstamm 415 (Institut für Seren und Impfstoffe "Metschnikov", Moskau)
  LD$_{50}$ (Maus, i.p.): $\leq 10^1$ Keime
  LD$_{50}$ (Maus, oral):~$10^3$ Keime
- Salmonella typhi Ty 2 Wildstamm (WHO-Referenzstamm) i.p. Infektion mit $10^6$ Keimen/Maus:
  • Persistenz im Leber-Milz-Homogenat:~19 Tage
  • Mittlere Keimzahl am 5. Tag nach Infektion $10 \times 10^4$ Keime pro Leber-Milz-Homogenat
- Salmonella typhi galE 21-Impfstamm von Germanier Dieser Vergleichs-Impfstamm besitzt kein Vi-Antigen i.p. Infektion mit $10^6$ Keimen/Maus:
  • Persistenz in Leber-Milz-Hemogenat:~2 Tage
- Hst-Mutanten vom Wildstamm und Impfstammen (Herstellung siehe DD 218 836)

Nährböden

- Nährager I und Nährbouillon I (SIFIN, Berlin- Weißensee)
- Minimalmedium (Mm):
  Modifizierte (Falkow, S., Rownd, R., Baron, L.S., J. Bacteriol. 84, 1303-1312 (1962)) M9-Salzlösung (Davis, B.O., Minigioli, E.S., J. Bacteriol. 60, 17-28 (1950)) mit 2,5% gewässertem Agar und 0,5% Dextrose für S.typhimurium sowie zusätzlich mit jeweils 20 $\mu$g Cystein(Ferak, Berlin und Thiamin (Serva, Heidelberg)/ml Minimalmedium für S.typhi.
  Diese Mm werden zur Vermehrung der Wildstämme eingesetzt.
- Substituiertes Mm Mm plus 100 $\mu$g Asparaginsäure (Reanal, Budapest)/ml.

Antibiotika und Tenside (Hersteller; Empfindlichkeit der Wildstämme in $\mu$g/ml)

- Walidixinsäure (Nevigramon, Ungarn; 6,2)
- Rifampicin (UMB, Bukarest; 12,5)
- Oleandemycin (Mederport, UdSSR; ≧100)
- Natrium-Dodecylsulfat (SDS; Serva, Heidelberg; ≧20 000)
- Natrium-Desoxycholat (SDO; VEB Berlin-Chemie; ≧10 000)

Tierversuche

1.) Salmonella typhimurium
- 16-18 Gramm schwere ICR-Mäuse (VEB Versuchstierproduktion Berlin-Schönewalde)
- Vergleichende Prüfung auf Virulenzverhalten und Immunogenität von potentiellen Impfstämmen ohne und mit anti-epidemischen Markern: i.p. und orale Applikation logarithmisch abgestufter Keimzahlen in 0,5 ml physiologischer Kochsalslösungen an jeweils 10 Mäuse
  - Virulenzverhalten: Bestimmung der Absterbeordnung innerhalb von 14 Tagen
  - Immunogenität: Belastung der die Immunisierung überlebenden bzw. tolerierenden Mäuse am 15. Tag i.p. mit $10^5$ Wildstammkeimen (>100 $LD_{50}$) in physiologischer Kochsalzlösung und Bestimmung der Anzahl der innerhalb von 2 Wochen sterbenden Tiere
- Bestimmung der Ausscheidungsdauer in den Faeces bei oral immunisierten Tieren: Täglicher Direktausstrich auf antibiotikahaltigem Selektivmedium (Impfstämme wurden mit Antibiotika-Resistenz-Marker als Erkennungs- und/oder Attenuierungsmarker versehen)

2.) Salmonella typhi
  a) Mäuse-Persistenz-Modell als indirektes Maß für die Attenuierung
  - 20-25 Gramm schwere NMRI-Auszucht-Mäuse
  - Nachweis der zeitlichen Persistenz von Wildstamm und den vom S.typhi Ty 2 abgeleiteten Mutanten in Mäusen, sowie maximale Keimzahl/Leber-Milz-Homogenat am 5. Tag nach Infektion:
    Die Mäuse werden mit $10^6$ (bei stärker attenuierten Stämmen zusätzlich $10^8$) Keimen (20 Stunden-Nähragarkultur, 37°C) i.p. infiziert. Beginnend am Tag nach der Infektion wurden täglich 2 Mäuse getötet, Leber und Milz unter sterilen Kautelen entnommen, mit Kieselgur zerrieben, anschließend in 10 ml physiologischer Kochsalzlösung aufgenommen und danach durch Ausspateln von 0,1 ml dieser Stammsuspension sowie von logarithmischen Verdünnungen auf Nähragar die Kolonie (Keim)-Zahl bestimmt. Die Nachweisgrenze dieser Methode liegt etwa bei 10 Keimen pro Leber-Milz-Homogenat.
    Durch Mittelwertsbildung aus 5 getrennten Ansätzen wird die Persistenzdauer (Persistenzkurve) und die mittlere Keimzahl am 5. Tag bestimmt, welche gut mit der Persistenzdauer korreliert. Als ermittelte Persistenzdauer in Tagen gilt der Zeitpunkt, an dem die Mehrzahl der Organhomogenate steril sind (einzelne Mäuse können bereits vorher "steril" sein bzw. nach diesem Zeitpunkt noch wenige Keime beherbergen).
  b) Chick embryo Test zum orientierenden Nachweis der Attenuierung (in Anlehnung an: Ketyi, I., Ann. Immunol. Hung. 9, 81-86 (1966))
    10-12 Tage alte Hühnerembryonen werden intravasal (über die freipräparierte Cherioallantois-Membran) mit geometrisch bzw. logarithmisch abgestuften Keimzahlen von Wildstamm und Mutanten in 0,1 ml physiologischer Kochsalzlösung infiziert. Nach 12-15 stündiger Bebrütung wird die Absterberate und daraus die $LD_{50}$ bestimmt.

Prüfung der Überlebensfähigkeit in der Außenwelt (Modellversuche)

- Leitungswasser und zur Orientierung
- Leitungswasser mit 0,5% Natrium-Dodecylsulfat (SDS)
- Leitungswasser mit 0,5% Natrium-Desoxycholat (SDO) wird mit $10^6$ Keimen/ml des Ausgangsstammes bzw. den von ihm abgeleiteten Klonen mit antiepidemischen Marker infiziert und täglich mittels Direktausstrich auf die entsprechenden lebenden Salmonella-Stämme hin untersucht. (Zwecks selektiver Isolierung und Identifizierung wurden diese Stämme mit einem zusätzlichen Antibiotika-Resistenz-Marker als Erkennungs- und/oder zugleich Attenuierungsmarker versehen).

Prüfung der Stabilität der Marker

- Asp$^-$-Marker:
  $10^8$ und $10^9$ Keime der Impfstämme werden auf Minimalmedium gespatelt und nach 48 sowie 72 Stunden Bebrütung bei 37°C die Zahl gewachsener Kolonien bestimmt Stabilitätsforderung:
  - Für Einmarker-Impfstämme: Reversionshäufigkeit $\leq 10^{-8}$ bzw. in vitro nicht nachweisbar
  - Für Mehrmarker-Impfstämme: Reversionshäufigkeit $\leq 10^{-7}$
- Hst- und Rbt- (sowie Rtt-)Marker
  siehe DD 218 836 A1 und DD 231 491 A1
- Rifampicin- (RNA-Polymerase) und Nalidixinsäure-Resistenz (Gyrase)
  siehe DD 155 294 A1

Beispiel 1

Herstellung von Asp$^-$-Mutanten - bei S.typhimurium ausgehend vom Wildstamm und bei S.typhi ausgehend von einer Hst-Mutante - und Auswahl eines geeigneten Klons:
Die Isolierung von Asparagin-auxotrophen Phänotypen erfolgt mit an sich bekannten Verfahren (MNG-Mutagenese, Penicilinscreening und replica plating; sinngemäß gilt ein Gleiches für Spontarmutanten sowie Transposon-Mutagenese).
Die bei diesem Vorgehen isolierten Asp$^-$-Klone wurde hinsichtlich ihrer Stabilität überprüft und alle Stämme mit einer Reversionshäufigkeit zur Protetrophie von $\leq 10^{-8}$ auf ihr Virulenzverhalten am i.p.-Mäusemodell getestet. Tabelle 1 (S.typhimurium) und Tabelle 2 (S.typhi) orientieren über die Zahl der stabilen Asp$^-$-Klone und ihr sich unter Vorbehalt auf wenigstens drei Gruppen verteilendes Virulenzverhalten.
Die Asparaginsäure-Auxotrophie-Mutanten mit einer in vitro nicht nachweisbaren bzw. $\leq 10^{-8}$ betragenden Reversion zur Prototrophie wie z.B.
- S.typhimurium asp$^-$23 $LD_{50}$ (Maus, i.p.)$\sim 10^{2,3}$ Keime
- S.typhimurium asp$^-$10 $LD_{50}$ (Maus, i.p.)$\sim 10^{4,3}$ Keime
- S.typhimurium asp$^-$15 $LD_{50}$ (Maus, i.p.)$\sim 10^{6,7}$ Keime
sind als Einmarker-Impfstämme für die Herstellung von Lebendimpfstoffen geeignet.
Für die in den nachfolgenden Beispielen beschriebene Herstellung der Zwei-, Drei- und Viermarker-Impfstämme wurde biespielsweise bei S.typhimurium der Klon Asp$^-$10 und für S. typhi die Hst/Asp$^-$6 Zweimarkermutante mit mäßig ausgeprägter Attenuierung verwendet. Sinngemäß läßt sich ein Gleiches mit Asp$^-$-Klonen geringerer oder höherer Attenuierung durchführen, um die für das Nutztier oder den Menschen angepaßte, optimale Attenuierungshöhe einzustellen.

Beispiel 2

Isolierung der Rbt-Mutanten:

Geeignete Hst-Klone von Wild- und Impfstämmen (Herstellang siehe: DD 218 836 A1 sowie Linde, K.,Arch. exper. Vet. med. 36, 657-662 (1982)) werden für 20 Stunden auf Nähragar kultiviert, anschließend in physiologischer Kochsalzlösung suspendiert, auf eine Keimzahl von $10^{10}$ und $10^9$ Keime/ml eingestellt und hiervon 0,1 ml - d.h. $10^9$ und $10^8$ Keime - auf Nähragar mit 5000 $\mu$g SDO/ml ausgespatelt. Gelleempfindlichkeit bzw. -toleranz wurde geprüft mit den Gallewirkstoff Desoxycholat. Die nach 48 stündiger Bebrütung bei 37°C gewachsenen Kolonien wurden auf einem Nähragar-Plattensatz mit Konzentrationen von
- 6 und 12 $\mu$g Oleandomycin/ml
- 600, 1200 und 2500 $\mu$g SDS/ml
- 5000 und 10 000 $\mu$g SDO/ml
geimpft and hinsichtlich vorhandenem Wachstum auf 5000 und 10 000 SDO/ml Nähragar, jedoch fehlendem Wachstum auf 12 $\mu$g Oleandomycin/ml Nährägar sowie 1200 $\mu$g SDS/ml Nähragar geprüft. Bei Klonen mit diesem Toleranz- bzw. Sensibilitätsverhalten handelt es sich um Rbt-Stämme.

Isolierung der Rtt-Mutanten:

In der Regel von Rbt-Klonen ausgehend werden $10^9$ und $10^8$ Keime dieses Stammes auf Nähragar mit 10 000 $\mu$g SDS/ml gespatelt. Die gewachsenen Kolonien werden auf Nähragar mit 10 000 und 20 000 $\mu$g SDS/ml und Nähragar mit 12 $\mu$g Oleandomycin/ml geimpft. Bei Klonen, welche auf dem Detergens-Medium, jedoch nicht auf dem Makrolid-Antibiotika-Medium wachsen, handelt es sich um Rtt-Revertanten.

Über das quantitative Sensibilitäts- bzw. Toleranzverhalten dieser Rbt- und Rtt-Klone - gegen Oleandomycin, SDO und SDS - im Vergleich zum Ausgangsstamm ohne und mit Hst-Marker orientiert Tabelle 3.

Die orientierenden Daten der Tabelle 3 bestätigen, daß nach Reversion zur Galle-Toleranz beim Rbt-Marker die hohe Sensibilität gegen (Anion)detergenzien und Makrolide weitgehend erhalten geblieben ist und den Werten des Hst-Ausgangsstammes entsprechen. (Sinngemäß gilt ein Gleiches für den Rtt-Marker, bei dem die hohe Sensibilität gegen Oleandomycin unverändert ist.)

### Beispiel 3

Nachweis der Stabilität des Rbt-Markers bzw. des Rtt-Markers:
Wird erbracht durch Spateln von $10^7$ bis $10^9$ Keimen auf Nähragar mit 60 $\mu$g Oleandomycin/ml sowie Nähregar mit 0,5% SDS (Rbt-Marker) bzw. nur auf Nähragar mit 60 $\mu$g Oleandomycin/ml (Rtt-Marker) und Bestimmung der Anzahl der nach 48 stündiger Bebrütung bei 37°C gewachsenen Kelonien. Die Reversionshäufigkeiten dar Rbt- und Rtt-Marker verschiedener Mutantenklene von S.typhimurium und S.typhi liegen zwischen ~$10^{-6}$ und ≤$10^{-8}$. Bei Verwendung dieser antiepidemischen Marker als zusätzliche optimierende Qualität für einen Impfstamm, sollte die Markerstabilität bei ≤$10^{-7}$ liegen.

### Beispiel 4

Überlebensfähigkeit von S.typhimurium (S.tm.) und S.typhi Ty 2 (S.Ty 2) ohne und mit antiepidemischen Marker (Stabilität ≤$10^{-8}$) in Leitungswasser ohne Zusätze sowie mit 0,5% SDO oder 0,5% SDS.

Wie Tabelle 4 zeigt, liegen beim Hst-, Rbt- und Rtt-Marker die Überlebenszeiten in Wasser bei ca. 50% des Wertes des Ausgangsstammes. Die zur Orientierung mitgeführten Werte der Überlebenszeiten in 0,5% SDO bzw. SDS zeigen, daß bei der Reversion zur Toleranz gegen Galle (Rbt) bzw. Galle und Aniondetergens (Rtt) die Resistenz der Ausgangsstämme ohne antiepidemischen Marker nicht voll erreicht wird.

### Beispiel 5

Vergleichende Charakterisierung des Virulenz (Attenuierungs)-Verhaltens und der Immunogenität von S.typhimurium Asp$^-$ (Mutantenklon mit mäßiger Attenuierung) ohne und mit Hst oder Rbt-Marker für Mäuse bei i.p. und oraler Immunisierung sowie i.p. Belastung am 15. Tag nach der"Immunisierung" mit $10^5$ Wildstamm-Keimen (>100ID$_{50}$).
Die Werte in Tabelle 5 zeigen, daß
- gemessen an der i.p. Absterbeordnung durch den zusätzlichen Einbau eines Hst- oder Rbt-Markers keine signifikante Verschiebung im Virulenzverhalten erfolgt;
- bei oraler Immunisierung die Hst-Mutante offensichtlich - als Folge der Inaktivierung eines Teiles der Mutantenkeime durch die Galle - "scheinbar" besser vertragen wird, aber als Folge der "scheinbar" (um den Faktor 10) geringeren Impfdosis entsprechend weniger immunogen ist. Demgegenüber zeigt die Rbt-Mutante etwa dem Ausgangsstamm ohne antiepidemischen Marker vergleichbare Werte.

### Beispiel 6

Einfluß des (Hst- und) Rbt-Markers auf das Persistenzverhalten in Tagen und die mittlere Keimzahl in Leber-Milz-Homogenat - als indirekten Maß für die Attenuierung bzw. Virulenzverhalten - von mit $10^6$ Keimen i.p. infizierten Mäusen.

Wie die Tabelle 6 ausweist, vermittelt die Hst-Mutation beim getesteten Klon Nr. 6 lediglich eine geringe (parenteral zum Tragen kommende) Attenuierung, sichtbar durch Verkürzung der Persistenzdauer von 19 auf 17 Tagen und eine etwa entsprechende Senkung der mittleren Keimzahlen am 5. Tag nach i.p. Infektion. Die Reversion ("Umschaltung") auf den Rbt-Marker hebt diese geringe Virulenzreduktion weitgehend wieder auf.

### Beispiel 7

Einfluß des (Hst- oder) Rbt-Markers auf die Ausscheidungsdauer in den Faeces bei einmaliger oraler Immunisierung von Mäusen mit $10^{10}$ Keimen von S.typhimurium-Impfstämmen ohne und mit dem antiepidemischen (Hst- oder) Rbt-Marker.
Wie Tabelle 7 zeigt, läßt sich beim - für diese Fragestellung infolge des Selektionsmarkers geeigneten - Direktausstrich der Einfluß des antiepidemischen Markers auf die Ausscheidungsdauer gut demonstrieren. Während der Impfstamm ohne antiepidemischen Marker bei der Mehrzahl der Mäuse über 12 Tage in den Faeces nachweisbar ist, sind Hst-Marker-Stämme nur 48 Stunden nachweisbar, während der Rbt-Stamm (in gewisser Übereinstimmung mit den Werten der Tabelle 4 sowie evtl. durch eine Sensibilität gegen flüchtige Fettsäuren) nur bzw. noch bis 5 Tage nachweisbar bleibt.

Beispiel 8

Abtrennung des Impfstammes (bzw. Wildstammes) ohne antiepidemischen Marker von solchen mit antiepidemischer Mutation:
Die Abtrennung erfolgt sinngemäß entsprechend Tabelle 3 durch Wachstum bzw. fehlende Vermehrung auf Nähragar mit 60 $\mu$g Oleandomycin/ml, Nähragar mit 0,5% Natrium-Desoxycholat und Nähragar mit 0,5% Natrium-Dodecylsulfat.

Beispiel 9

Einbau von weiteren attenuierenden Stoffwechseldrift-Markern, wie z.B. einer Rifampicin-Resistenz-(RNA-Polymerase)-Mutation bzw. Nalidixinsäure-Resistenz-(Gyrase)-Mutation mit zusätzlicher Attenuierung, welche am i.p.-Mäusemodell die $LD_{50}$-Werte des S.typhimurium Zweimarkerstammes $Asp^-10$ Hst bzw. Rbt von ~$10^5$ Keime auf $10^6$ bis $10^7$ Keime (bzw. einen wünschenswert niedrigeren oder höheren Wert anhebt und die Persistenz des S.typhi Hst/$Asp^-$ von 12 Tagen auf 9 bis 3 Tage (oder evtl. eine noch geringere Zeit) verkürzt:

S.typhimurium

Von den oben beschriebenen S.tm. $Asp^-10$ Hst bzw. Rbt Zweimarker-Stämmen wurde, wie in DD 155 294 A1 beschrieben, mittels spontaner Mutation Rifampicin-Resistenzmutanten bzw. Nalidixinsäuremutanten isoliert and am i.p.-Mäusemodell diejenigen Klone ausgelesen und überprüft, welche den i.p. $LD_{50}$-Wert der S.tm. $Asp^-10$ Hst bzw. Rbt Zweimarkerstämme von ~$10^5$ Keimen um 1-2 log-Stufen anhoben.
Über das i.p. Virulenzverhalten und die Immunogenität der auf diese Weise hergestellten Dreimarker-Impfstämme sowie der Vorläufer-Impfstämme $Asp^-10$ und $Asp^-10$/Hst bzw. $Asp^-10$/Rbt orientiert Tabelle 8.
Die in Tabelle 8 aufgeführten Zwei- und Dreimarker-Impfstämme sind - als Beispiel für weitere noch gleichem Schema mit anderen $Asp^-$-Ausgangsklonen (gleicher, geringerer oder höherer Attenuierung) aufgebauten Mehrmarker-Impfstämmen - für die Herstellung von Lebendimpfstoffen geeignet.

S.typhi - Herstellung eines geeigneten Dreimarker-Impfstammes:

Von dem oben beschriebenen S.typhi Ty 2 Hst/$Asp^-6$-Zweimarkerstamm wurde, wie in DD 155 294 A1 beschrieben, mittels spontaner Mutation Rifampicin-Resistenzmutanten isoliert und am i.p.-Mäuse-Persistenz-Modell diejenigen Klone ausgelesen und überprüft, welche eine zusätzliche Verkürzung der Persistenzdauer um mehrere Tage zeigten.
Wie die Tabelle 9 ausweist, läßt sich i.p.-Mäusemodell die stufenweise zunehmende Virulenzreduktion deutlich belegen mit Hilfe des Persistenzverhaltens und der mittleren Keimzahl 5 Tage nach der Infektion, während der chick embryo-Test lediglich orientierende Hinweise vermittelt.
Der aufgeführte Viermarker-Impfstamm S.typhi Ty 2 Rbt/$Asp^-$/Rif/Nal 18 wurde bewußt in seiner Attenuierungshöhe als etwas über der Virulenzreduktion des S.typhi galE 21a (Germanier) liegend ausgewählt (und kann bei Bedarf gegen Klone mit solchem Werten ausgetauscht werden, welche exakt denen des Germanier-Impfstammes entsprechen, oder eine höhere Attenuierung zeigen).

Beispiel 10

Herstellung von S.typhimurium $Asp^-$/Stoffwechseldrift-Zweimarker-Impfstämmen zur Verwendung als Lebendimpfstoff. In einen geeigneten $Asp^-$-Klon (ohne antiepidemischen Marker) wird entsprechend Beispiel 9 eine geeignete Stoffwechseldrift-Mutation eingebaut.
(Es ist auch möglich solche Zweimarker-Impfstämme dadurch zu erhalten, indem man von einem unter Beispiel 3 beschriebenen Dreimarker-Impfstamm den antiepidemischen Marker experimentell zur Wildtyp-Toleranz revertiert.)

Beispiel 11

Herstellung von S.typhimurium-Stoffwechseldrift-Zwei- und Dreimarker-Impfstämmen zur Verwendung als Lebendimpfstoff.

In den S.typhimurium-Wildstamm werden entsprechend Beispiel 9 schrittweise geeignete Stoffwechseldrift-Mutationen eingebaut, wie z.B. eine Nalidixinsäure-Resistenz (Gyrase)-Mutation im ersten und eine Bifampicin-Resistenz (RNA-Polymerase)-Mutation im zweiten Schritt. Der Einbau der dritten schwach bzw. mäßig virulenzreduzierenden Streptomycin-Resistenz (Ribosomen-Protein)-Mutation erfolgt durch Herstellung spontaner chromosonaler Resistenz-Mutanten auf Nähragar mit $\geq$400 $\mu$g Streptomycin/ml mit nachfolgender Auslose und Überprüfung derjenigen Klone am i.p. Mäusemodell, welche den i.p. $LD_{50}$-Wert der S.typhimurium Nal/Rif Zweimarkerstämme um weitere 1 bis 3 logarithmische Stufen (oder jedem anderen gewünschten Wert) anheben (siehe Tabelle 10).

Die in Tabelle 10 aufgeführten (Ein)-, Zwei- und Dreimarker-Impfstämme sind - als Beispiel für weitere nach gleichem Schema und auch mit anderen als den angeführten Stoffwechseldrift-Markern aufgebauten Impfstämmen - für die Herstellung von Impfstoffen geeignet.

Beispiel 12

Vorscreening auf die vermutliche Attenuierungshöhe bei Stoffwechseldrift-Mutanten auf Grundlage der linearen Besiehungen zwischen der Generationszeit (gemessen mit dem Bakterien-Testsystem MS 2 (Abbott) oder einem anderen geeigneten Testsystem) und dem Logarithmus der $LD_{50}$ (Maus, i.p.) dargestellt in Abbildung 1 mit z.B. S.typhimurium. (Bei Salmonellan ohne ausgeprägte Mäusevirulenz, wie z.B. S.abortus ovis ist dieser Vorscreening eine vorteilhafte Methode für die engere Auswahl geeigneter Impfstamm-Klone vor ihrer Prüfung am Nutztier.)

Beispiel 13

Die Abtrennung der Salmonella Impfstämme von Wildstämmen erfolgt wie in Tabelle 11 ausgewiesen.

Beispiel 14

Massenkultur, Präparation und Anwendung der Impfstämme: Zur Herstellung des Lebendimpfstoffes werden die Impfstämme z.B. in einem halbsynthetischen Nährmedium bei 37°C bis zum Ende der logarithmischen Phase im Fermenter gezüchtet. Die Bakteriensuspensionen werden als Lyophilisat (oder einer anderen geeigneten Form, wie z.B. Flüssigkeitsdosen, Dragee) präpariert. Der so erhaltene Impfstoff wird in der Regel als einmalige Dosis von $10^7$ bis $10^{10}$ Lebendkeimen an Nutztiere oral (evtl. parenteral) bzw. oral an Menschen verabreicht.

An Stelle einer zwei- bis dreimaligen Applikation eines an der Grenze zur Überattenuierung sich bewegenden Impfstammes bestehen die Alternativen

- Einsatz eines etwas geringer attenuierten Impfstammes, oder
- Erstimmunisierung mit einem er der Grenze zur Überattenuierung sich bewegenden Impfstamm und Booster nach angemessener Zeit mit einem Impfstemm geringerer Attenuierungshöhe.

Tabelle 1

Salmonella typhimurium: Einfluß der Asparaginsäure-Auxotrophie auf das Virulenzverhalten
Getestet in zwei getrennten Versuchen bei neun Klonen (Genotypen mit einer Reversionshäufigkeit $\leq 10^{-8}$) am i.p.-Mäusemodell

| Asp⁻-Klon Nummer | i.p.$LD_{50}$ ICR-Laus Versuch A | Versuch B |
|---|---|---|
| | Keimzahl $10^x$ | |
| 23 | 2,0 | 2,5 |
| 10 | 4,3 | 4,3 |
| 12 | 4,6 | 4,3 |
| 14 | 5,5 | 5,5 |
| 15 | 6,8 | 6,5 |
| 13 | 6,5 | 7,5 |
| 16 | 7,0 | 7,0 |
| 18 | 7,5 | 7,5 |
| 11 | 8,0 | 7,0 |
| Wildstamm | $\leq 1$ | $\leq 1$ |

Tabelle 2

S.typhi Ty 2: Einfluß der Asparaginsäure-Auxotrophie auf die Persistenzdauer, die mittlere Keimzahl (KZ) im Leber-Milz-Homogenat am 5.Tag nach Infektion und die $LD_{50}$ (Beobachtungszeitraum: |——— 24 Stunden) bei sieben Asp⁻-Klonen (Reversionshäufigkeit $\leq 10^{-8}$) einer S.typhi Ty 2-Hst-Mutante und einer S.typhi Ty 2-Rbt-Mutante am i.p.-Mäusemodell

| S.typhi Ty 2 (mögliche Attenuierungs-gruppe) | Persistenz in Tagen | mittl. KZ am 5. Tag | i.p. $LD_{50}$ Keimzahl |
|---|---|---|---|
| Wildstamm | 19 | $10 \times 10^4$ | $5 \times 10^6$ |
| Hst-Mutante | 17 | $6 \times 10^4$ | $1 \times 10^7$ |
| Rbt-Mutante | 18 | $8 \times 10^4$ | $1 \times 10^7$ |
| Gruppe I: Hst/Asp⁻11 | 15 | $5 \times 10^4$ | $1 \times 10^8$ |
| Gruppe II: Hst:Asp⁻16 | 14 | $3 \times 10^4$ | $2 \times 10^8$ |
| Hst/Asp⁻10 | 14 | $2 \times 10^4$ | $1 \times 10^8$ |
| Hst/Asp⁻14 | 12 | $2 \times 10^4$ | $1 \times 10^8$ |
| Hst/Asp⁻13 | 12 | $2 \times 10^4$ | $2 \times 10^8$ |
| Hst/Asp⁻ 6 | 12 | $1 \times 10^4$ | $2 \times 10^8$ |
| Gruppe III: Hst/Asp⁻15 | 5 | $4 \times 10^1$ | $1 \times 10^9$ |

Tabelle 3

Vergleichende Charakterisierung der Sensibilität bzw. Toleranz gegen Oleandomycin, SDO und SDS von S.typhimurium (S.tm.) und S.typhi Ty 2 (S.Ty 2) ohne und mit Hst- bzw. Rbt- bzw. Rtt-Marker

| Stämme | antiepi-demischer Marker | Wachstum auf Nähragar mit ... µg Noxe/ml | | | | | | | | |
| | | Oleandomycin | | | SDO | | | SDS | | |
| | | 6 | 12 | 100 | 1200 | 2500 | 10000 | 600 | 2500 | 20000 |
| S.tm. | ø | + | + | + | + | + | + | + | + | + |
| | Hst | (+) | ø | ø | ø | ø | ø | ø | ø | ø |
| | Rbt | + | ø | ø | + | + | (+) | (+) | ø | ø |
| | Rtt | + | ø | ø | + | + | + | + | + | (+) |
| S.Ty 2 | ø | + | + | + | + | + | + | + | + | + |
| | Hst | (+) | ø | ø | ø | ø | ø | ø | ø | ø |
| | Rbt | + | ø | ø | + | + | (+) | (+) | ø | ø |
| | Rtt | + | ø | ø | + | + | + | + | + | (+) |

Tabelle 4

S.typhimurium und S.typhi Ty 2 ohne und mit antiepidemischen Markern:

Durchschnittliche Überlebenszeit in Tagen von $10^6$ Keimen/ml Leitungswasser ohne oder mit 0,5% SDO oder 0,5% SDS

| $10^6$ Keime: Leitung-wasser | Überlebenszeit in Tagen in/von | | | | | | | |
| | S.typhimurium | | | | S.typhi Ty 2 | | | |
| | ø | Hst | Rbt | Rtt | ø | Hst | Rbt | Rtt |
| ø | 20 | 8 | 10 | 12 | 7 | 2 | 3 | 4 |
| SDO | 15 | <1 | 6 | 9 | 5 | <1 | 2 | 3 |
| SDS | 12 | <1 | 3 | 4 | 4 | <1 | 1 | 1 |

16

Tabelle 5

Attenuierung und Immunogenität von S.typhimurium (S.tm.) Asp⁻ und der von diesem Einmarkerstamm abgeleiteten Zweimarker-Mutanten mit Hst-bzw. Rbt-Marker (i.p. sowie orale Immunisierung von je 10 Mäusen mit $10^x$ Mutantenkeimen; i.p. Belastung der die orale Immunisierung überlebenden Tiere am 15.Tag mit $10^5$ Wildstamm-Keimen ( > 100 $LD_{50}$)

| S.tm. | i.p.<br>$10^x$ | "Immunis."<br>✝/10 | oral<br>$10^x$ | Immunis.<br>✝/10 | Belastg.<br>✝/N |
|---|---|---|---|---|---|
| Asp⁻ | 3 | 2 | 5 | 0 | 4/10 |
| | 4 | 5 | 6 | 0 | 2/10 |
| | 5 | 8 | 7 | 1 | 1/9 |
| | 6 | 10 | 8 | 2 | 0/8 |
| Asp⁻/<br>Hst | 3 | 2 | 5 | 0 | 5/10 |
| | 4 | 4 | 6 | 0 | 3/10 |
| | 5 | 7 | 7 | 0 | 2/10 |
| | 6 | 9 | 8 | 0 | 2/10 |
| Asp⁻/<br>Rbt | 3 | 3 | 5 | 0 | 3/10 |
| | 4 | 3 | 6 | 0 | 3/10 |
| | 5 | 7 | 7 | 0 | 1/10 |
| | 6 | 10 | 8 | 2 | 0/8 |

Tabelle 6

Salmonella typhi Ty 2 (S.Ty 2): Einfluß des Hst- und Rbt-Markers auf das Persistenzverhalten in Tagen und die mittlere Keimzahl (KZ) in Leber-Milz-Homogenat am 5.Tag nach der Infektion am i.p. Mäusemodell

| S.Ty 2 | i.p. $10^6$ Keime/Maus Persistenz in Tagen | mittl. KZ am 5. Tag |
|---|---|---|
| Ausgangs- stamm | 19 | $10 \times 10^4$ |
| Hst 6 | 17 | $6 \times 10^4$ |
| Rbt 31 | 18 | $8 \times 10^4$ |
| Rbt 32 | 18 | $10 \times 10^4$ |

Tabelle 7

Einfluß des Hst- bzw. Rbt-Markers auf die Ausscheidungsdauer in Faeces von S.typhimurium (S.tm)-Impfstämmen bei Mäusen nach einmaliger oraler Gabe von $10^{10}$ Keimen

(Zwecks optimalem Nachweis im Direktausstrich sind ausgewählte Stämme mit Selektions- und zugleich Attenuierungsmarker versehen.)

| S.tm.- Impfstamm | Impfstamm-Mutanten-Nachweis in Faeces bei je 5 Mäusen am 1.-12.Tag nach oraler Immunisierung mit ~ $10^{10}$ Keimen | | | | | | | | |
|---|---|---|---|---|---|---|---|---|---|
| | 1 | 2 | 3 | 4 | 5 | 6 | 8 | 10 | 12 |
| Asp⁻/Rif | ──────────────────────────────────────▶ | | | | | | | | |
| Asp⁻/Rif/Hst | ──▶ | | | | | | | | |
| Asp⁻/Nal/Rbt | ──────────▶ | | | | | | | | |

18

Tabelle 8

Vergleichende Charakterisierung der Attenuierungshöhe und Immunogenität von Einmarker-Impfstamm S.tm Asp⁻10, der Zweimarker-Impfstämme S.tm Asp⁻10/Hst bzw. Rbt sowie den Dreimarker-Impfstämmen S.tm Asp⁻/Rbt/Nal 19; Asp⁻10/Hst/Rif 10 und Asp⁻/Hst/Rif 24 am i.p. Mäusemodell

| S.typhimurium Ein- | Zwei- | Drei Markerstamm | i.p.LD$_{50}$ Maus $10^X$ Keime | i.p.Immunis. mit $10^X$ Keimen | Schutzwert (i.p.Belastg. am 15. Tag mit $> 100$ LD$_{50}$ |
|---|---|---|---|---|---|
| Asp⁻10 | | | 4,3 | 2 | ∼ 90% |
| Asp⁻10 | Hst | | 5,0 | 3 | ∼ 95% |
| Asp⁻10 | Rbt | | 5,0 | 3 | ∼ 95% |
| Asp⁻10 | Rbt | Nal 19 | 6,0 | 4 | ∼ 95% |
| Asp⁻10 | Hst | Rif 10 | 6,5 | 4 | ∼ 95% |
| Asp⁻10 | Hst | Rif 24 | 7,0 | 5 | ∼ 95% |
| Wildstamm-Kontr. | | | $\leq 1,0$ | – | 0% |

Tabelle 9

Vergleichende Charakterisierung von S.typhi Ty 2-Ausgangsstamm und seinen in direkter Reihenfolge abgeleiteten Ein- und Zweimarker-Stämmen, den Drei- und Viermarker-Impfstämmen (mit abgestufter Attenuierungshöhe) sowie dem international eingeführten Impfstamm S.typhi gal-E 21a (Germanier)

(Persistenzdauer in Tagen, mittlere Keimzahl/Leber-Milz-Homogenat (KZ) am 5.Tag nach i.p. Infektion der Mäuse mit $10^6$ und $10^8$ Keimen sowie $LD_{50}$ im chick embryo-Test.)

| Salmonella typhi Ty 2 | | | | ip $10^6$ Keime | | ip $10^8$ Keime | | chick embryo $LD_{50}$ |
| Ein- | Zwei- | Drei- | Vier- | Pers. | KZ am | Pers. | KZ am | |
| Marker-Impfstämme | | | | Tag | 5.Tag | Tage | 5.Tag | Keime |
|---|---|---|---|---|---|---|---|---|
| Wildstamm Ty 2 | | | | 19 | $1 \times 10^5$ | 100%† | | 1-5 |
| Hst6 | | | | 17 | $6 \times 10^4$ | 100%† | | 1-5 |
| Rbt | | | | 18 | $8 \times 10^4$ | 100%† | | 1-5 |
| Hst | Asp⁻6 | | | 12 | $1 \times 10^4$ | 100%† | | 5-10 |
| Hst | Asp⁻ | Rif 9 | | 9 | $2 \times 10^2$ | 12 | 30%† $3 \times 10^4$ | 100 |
| Rbt | Asp⁻ | Rif | Nal 1 | 6 | $10^1$ | 10 | $2 \times 10^3$ | nicht gepr. |
| Rbt | Asp⁻ | Rif | Nal18 | 3 | $10^1$ | 8 | $3 \times 10^2$ | $10^7$ |
| S.typhi gal-E 21a | | | | 2 | $10^1$ | 6 | $7 \times 10^1$ | $10^4$ |

Tabelle 10

Vergleichende Charakterisierung der Attenuierungshöhe und Immunogenität von Stoffwechseldrift-(Ein-) Zwei- und Dreimarker-Impfstämmen von S.typhimurium am i.p. Mäusemodell

Die Mutanten werden hergestellt über den schrittweisen Einbau von gering bis mäßig virulenzreduzierenden Stoffwechseldrift-Mutationen, wie z.B. einer Nalidixinsäure-Resistenz-(Gyrase-)Mutation als ersten Marker, einer Rifampicin-Resistenz-(RNA-Polymerase-)Mutation als zweiten Marker und einer Streptomycin-Resistenz-(Ribosomenprotein-)Mutation als dritten Marker.

| S.typhimurium Ein-, Zwei-, Drei- Marker-Stamm | i.p.LD$_{50}$ $10^x$ Keime | i.p. Immuni- sierung mit $10^x$Keimen | Schutzwert (i.p.Belastg. am 15. Tag mit $> 100$ LD$_{50}$) |
|---|---|---|---|
| Nal 2 | 3,5 | ./. | ./. |
| Nal 2 Rif 10 | 7,0 | 4 | 95% |
| Nal 2 Rif 1 | 5,0 | 3 | 95% |
| Nal 2 Rif 1 Sm 1 | 6,5 | 4 | 95% |
| Nal 2 Rif 1 Sm 2 | 8,0 | 5 | 95% |
| Wildstamm-Kontr. | $\leq 1$ | ./. | 0% |

21

**Tabelle 11**

Abtrennung der Salmonella-Impfstämme von Wildstämmen durch nachweisbares oder fehlendes Wachstum auf unterschiedlichen Test-Nährböden

| Wachstum auf bezügl.: Einzelmarker | NA | 100 µg Rifampicin | 50 µg Nalidixins. | 100 µg Streptomyc. | 60 µg Oleandomyc | 0,5% Dodecylsulfat | 0,5% Desoxycholat | Mm |
|---|---|---|---|---|---|---|---|---|
| Hst | + | ø | ø | ø | ø | ø | ø | + |
| Rbt | + | ø | ø | ø | ø | ø | + | + |
| Rtt | + | ø | ø | ø | ø | + | + | + |
| Asp⁻ | + | ø | ø | ø | + | + | + | ø |
| Rif | + | + | ø | ø | + | + | + | + |
| Nal | + | ø | + | ø | + | + | + | + |
| Sm | + | ø | ø | + | + | + | + | + |
| z.B.Markerkombination | | | | | | | | |
| Asp⁻/Hst | + | ø | ø | ø | ø | ø | ø | ø |
| Asp⁻/Rbt | + | ø | ø | ø | ø | ø | + | ø |
| Asp⁻/Rif | + | + | ø | ø | + | + | + | ø |
| Asp⁻/Rif/Nal | + | + | + | ø | + | + | + | ø |
| Asp⁻/Rif/Nal/Rbt | + | + | + | ø | ø | ø | + | ø |
| Rif/Nal/Sm | + | + | + | + | + | + | + | + |
| Wildstamm | + | ø | ø | ø | + | + | + | + |

NA = Nähragar; Mm = Minimalmedium

**Patentansprüche**

1.  Salmonella-Lebendimpfstoff mit Wirtsspezies-angepaßter Attenuierungshöhe und mit antiepidemischer Potenz gegen Salmonella-Infektionen, gekennzeichnet dadurch, daß der Lebendimpfstoff einen aus Hst-Klonen gewonnenen Galletoleranten revertanten (Rbt-Stamm) oder einen aus Rbt-Klonen gewonnenen Tensid-toleranten revertanten (Rtt-Stamm) Salmonella-Lebendimpfstamm enthält.

2. Impfstoff nach Anspruch 1, gekennzeichnet dadurch, daß der Lebendimpfstoff als weitere Marker mindestens einen virulenzreduzierenden Stoffwechseldrift-Marker und/oder einen virulenzreduzierenden Asparaginsäure-Auxotrophie-Marker enthält.

3. Verfahren zur Herstellung eines Salmonella-Lebendimpfstoffes mit Wirtsspezies-angepaßter Attenuierungshöhe und mit antiepidemischer Potenz gegen Salmonella-Infektionen, gekennzeichnet dadurch, daß mittels spontaner Mutation aus Makrolid-, Galle- und Anion-Detergent-sensitiven Hst-Klonen unter Verwendung von Galle-haltigem Nähragar Galle-tolerante Rbt-Klone und aus diesen gegebenenfalls unter Verwendung von Anion-Detergent-haltigem Nähragar Tensid-tolerante Rtt-Klone isoliert werden, die erhaltenen Rbt- bzw. Rtt-Klone in immunogene Impfstämme oder in einen für die Impfstamm-Entwicklung vorgesehenen Wildstamm oder in als Zwischenprodukt vorliegende Ein-, Zwei- oder Mehrmarker-Impfstämme mit graduell abgestufter Attenuierung eingebaut werden und aus den erhaltenen Stämmen mittels an sich bekannter Verfahren Lebendimpfstoffe hergestellt werden.

4. Verfahren nach Anspruch 3, gekennzeichnet dadurch, daß zur Isolierung von Rbt-Mutanten Hst-Klone von Wild- und Impfstämmen 20 Stunden auf Nähragar kultiviert werden, anschließend in physiologischer Kochsalzlösung suspendiert werden, diese Suspension auf eine Keimzahl von $10^{10}$ und $10^9$ Keime/ml eingestellt wird und von den eingestellten Suspensionen 0,1 ml auf Nähragar mit 5000 $\mu$l Natrium-Desoxycholat/ml ausgespatelt werden, nun der beimpfte Nähragar 48 Stunden bei 37°C bebrütet wird und die gewachsenen Kolonien auf einem Nähragar-Plattensatz mit Konzentrationen von
- 6 und 12 $\mu$g Oleandomycin/ml
- 600, 1200 und 2500 $\mu$g Natrium-Dodecylsulfat/ml
- 5000 und 10000 $\mu$g Natrium-Desoxycholat/ml
geimpft werden, wobei die Rbt-Stämme ein Toleranz- bzw. Sensibilitätsverhalten aufweisen, das auf 5000 und 10000 $\mu$g Natrium-Desoxycholat/ml Nähragar zu einem Wachstum führt und auf 12 $\mu$g Oleandomycin/ml Nähragar sowie 1200 $\mu$g Natrium-Dodecylsulfat/ml Nähragar durch fehlendes Wachstum gekennzeichnet ist.

5. Verfahren nach Anspruch 3, gekennzeichnet dadurch, daß zur Isolierung von Rtt-Mutanten Rbt-Klone mit $10^9$ und $10^8$ Keime dieses Stammes auf Nähragar mit 10000 $\mu$g Natrium-Dodecylsulfat/ml gespatelt werden, die gewachsenen Kolonien auf Nähragar mit 10000 und 20000 $\mu$g Natrium-Dodecylsulfat/ml und Nähragar mit 12 $\mu$g Oleandomycin/ml geimpft werden, wobei die Rtt-Stämme auf dem Detergent (und Galle)-Medium, jedoch nicht auf dem Makrolid-Antibiotika-Medium wachsen.

6. Salmonella-Lebendimpfstoff gemäß einem der Ansprüche 1 und 2 zur Immunisierung von Menschen und Nutztieren gegen Salmonella-Infektionen.

**Claims**

1. A live salmonella vaccine having a host species-adapted attenuation level and an anti-epidemic potency against salmonella infections, characterized in that the live vaccine contains a gall-tolerant revertant (Rbt strain) salmonella live vaccine strain prepared from Hst clones or a tenside-tolerant revertant (Rtt strain) salmonella live vaccine strain prepared from Rbt clones.

2. The vaccine according to claim 1, characterized in that the live vaccine contains as further marker at least one virulence reducing metabolism drift marker and/or a virulence reducing asparaginic acid-auxotrophy marker.

3. A method for the production of a live salmonella vaccine with a host species-adapted attenuation level and with an anti-epidemic potency against salmonella infections, characterized by isolating, by means of spontaneous mutation, gall-tolerant Rbt clones from macrolide-sensitive, gall-sensitive and anion-detergent-sensitive Hst clones with the use of gall-containing nutrient agar and, possibly with the use of anion-detergent-containing nutrient agar, isolating tenside-tolerant Rtt clones from the gall-tolerant Rbt clones, incorporating the obtained Rbt clones or Rtt clones into immunogenic vaccine strains or into a wild strain provided for the vaccine strain development or into one marker, two markers or multiple markers vaccine strains with gradually stepped attenuation which are present as intermediate product, and producing live vaccine from the obtained strains by means of methods known per se.

4. The method according to claim 3, characterized by cultivating Hst clones of wild strains and vaccine strains for 20 h on nutrient agar for the isolation of Rbt mutants, subsequently suspending the same in physiological salt solution, adjusting this suspension to a germ number of $10^{10}$ and $10^9$ germs/ml, and spattling 0,1 ml of the adjusted suspensions onto nutrient agar with 5000 $\mu$l sodium desoxycholate/ml, now breeding the vaccinated nutrient agar for 48 h at 37° C, and vaccinating the grown colonies on a nutrient agar plate set with concentrations of
   - 6 and 12 $\mu$g oleandomycine/ml
   - 600, 1200 and 2500 $\mu$g sodium-dodecylsulfate/ml
   - 5000 and 10000 $\mu$g sodium-desoxycholate/ml

   wherein the Rbt strains have a tolerance or sensibility behaviour which results in a growth on 5000 and 10000 $\mu$g sodium-desoxycholate/ml nutrient agar and which is characterized by missing growth on 12 $\mu$g oleandomycine/ml nutrient agar and 1200 $\mu$g sodium-dodecylesulfat/ml nutrient agar.

5. The method according to claim 3, characterized by spattling Rbt clones with $10^9$ and $10^8$ germs of this strain onto nutrient agar with 10000 $\mu$g sodium-dodecylsulfate/ml for the isolation of Rtt mutants, vaccinating the grown colonies on nutrient agar with 10000 and 20000 $\mu$g sodium-dodecylsulfat/ml and nutrient agar with 12 $\mu$g oleandomycine/ml, wherein the Rtt strains grow on the detergent (and gall)-medium, however, not on the macrolide-antibiotics-medium.

6. The salmonella live vaccine according to one of the claims 1 and 2 for the immunization of human beings and animals against salmonella infections.

## Revendications

1. Vaccin vivant de salmonella ayant un degré d'atténuation adapté à l'espèce hôte et une potentialité anti-épidémique contre des infections provoquées par les salmonella, caractérisé en ce qu'il contient une souche de vaccin vivant de salmonella révertante, tolérant la bile et obtenue à partir de clones de Hst (souche Rbt) ou une souche de vaccin vivant de salmonella révertante, tolérant les agents tensio-actifs et obtenue à partir de clones de Rbt (souche Rtt).

2. Vaccin selon la revendication 1, caractérisé en ce que le vaccin vivant contient, comme autres marqueurs, au moins un marqueur de déplacement métabolique réducteur de virulence et/ou un marqueur d'auxotrophie à l'acide aspartique réducteur de virulence.

3. Procédé d'obtention d'un vaccin vivant de salmonella ayant un degré d'atténuation adapté à l'espèce hôte et une potentialité anti-épidémique contre des infections provoquées par les salmonella, caractérisé en ce qu'au moyen d'une mutation spontanée à partir de clones de Hst sensibles aux macrolides, à la bile et aux détergents anioniques, en utilisant un milieu nutritif d'agar contenant de la bile, on isole des clones de Rbt tolérant la bile, et à partir de ceux-ci, éventuellement en utilisant un milieu nutritif d'agar contenant un détergent anionique, on isole des clones de Rtt tolérant les agents tensio-actifs, on introduit les clones de Rbt ou les clones de Rtt dans des souches immunogènes d'inoculation, ou dans une souche sauvage prévue pour le développement de la souche d'inoculation, ou dans des souches d'inoculation à mono-, bi- ou polymarqueurs, qui sont présentes en tant que produit intermédiaire et possèdent une atténuation échelonnée, et, à partir des souches obtenues, on prépare des vaccins vivants au moyen de procédés connus en soi.

4. Procédé selon la revendication 3, caractérisé en ce que, pour isoler des mutants de Rbt, on cultive des clones de Hst provenant de souches sauvages et de souches d'inoculation, pendant 20 heures, sur un milieu nutritif d'agar, ensuite, on les met en suspension dans du sérum physiologique, on règle le nombre de germes de cette suspension à $10^{10}$ et $10^9$ germes/ml, on étale 0,1 ml des suspensions ajustées sur un milieu nutritif d'agar avec 5 000 $\mu$l de désoxycholate de sodium/ml, ensuite on fait incuber l'agar nutritif inoculé, à 37° C, pendant 48 heures, et on inocule les colonies formées, sur des plaques d'agar nutritif, avec des concentrations de
   - 6 et 12 $\mu$g d'oléandomycine/ml,
   - 600, 1 200 et 2 500 $\mu$g de dodécylsulfate de sodium/ml,

- 5 000 et 10 000 $\mu$g de désoxycholate de sodium/ml,

les souches Rbt ayant un comportement de tolérance et de sensibilité qui conduit à leur croissance sur 5 000 et 10 000 $\mu$g de désoxycholate de sodium/ml d'agar nutritif, et qui est caractérisé par une absence de croissance sur agar nutritif à 12 $\mu$g d'oléandomycine/ml ainsi que sur agar nutritif à 1 200 $\mu$g de dodécylsulfate de sodium/ml.

5.  Procédé selon la revendication 3, caractérisé en ce que, pour isoler des mutants de Rtt, on étale des clones de Rbt, avec $10^9$ et $10^8$ germes de cette souche, sur un milieu nutritif d'agar contenant 10 000 $\mu$g de dodécylsulfate de sodium/ml, on inocule les colonies formées sur l'agar nutritif avec 10 000 et 20 000 $\mu$g de dodécylsulfate de sodium/ml et de l'agar nutritif avec 12 $\mu$g d'oléandomycine/ml, les souches Rtt se développant sur le milieu à base de détergent (et de bile), mais pas sur le milieu à base d'antibiotiques du type macrolides.

6.  Vaccin vivant de salmonella selon l'une des revendications 1 et 2 pour l'immunisation de l'homme et des animaux de rendement contre des infections provoquées par les salmonella.

Abb.1